# EUROPÄISCHE PATENTSCHRIFT

(11) **EP 0 690 046 B1**
(45) Veröffentlichungstag und Bekanntmachung des Hinweises auf die Patenterteilung: **23.12.1998**
(21) Anmeldenummer: 95109783.1
(22) Anmeldetag: 23.06.1995
(51) Int. Cl.: C07C 253/30, C07C 255/50, C07C 45/68, C07C 47/546, C07C 41/30, C07C 43/205, C07C 17/26, C07C 49/784

(54) **Verfahren zur Herstellung von Biphenylen mit Palladacyclen als Katalysatoren**
Method of preparation of biphenyls using palladacyclen as catalyst
Procédé pour la préparation des biphényles avec palladacyclen comme catalysateur

(30) Priorität: 01.07.1994 DE 4423061
(43) Veröffentlichungstag der Anmeldung: 03.01.1996
(73) Patentinhaber: Clariant GmbH, 65929 Frankfurt am Main (DE)
(72) Erfinder: Beller, Matthias, Dr., D-65510 Idstein (DE); Herrmann, Wolfgang Anton, Prof. Dr., D-85354 Freising (DE); Brossmer, Christoph, Dr., D-60318 Frankfurt (DE)

(56) Entgegenhaltungen:
- EP-A- 0 470 795
- WO-A-91/09008
- WO-A-94/00423
- US-A- 5 254 776

## Beschreibung

Die vorliegende Erfindung betrifft ein neues Verfahren zur Herstellung von Biphenylen aus Arylhalogeniden oder Arylsulfonaten und Arylboronsäuren mit neuartigen Katalysatoren, sogenannten Palladacyclen.

Biphenyle haben technische Bedeutung als Feinchemikalien für Flüssigkristalle und verwandte Anwendungen und als Ausgangsprodukte für Wirkstoffvorprodukte, insbesondere als Angiotensin-Antagonisten.

Eine häufig angewandte Methode zur Synthese von Biphenylen im Labormaßstab ist die palladiumkatalysierte Kreuzkupplung (Suzuki-Kupplung), bei der Iod-, Bromaromaten oder Arylsulfonate mit metallorganischen Arylderivaten, insbesondere Arylborderivaten in Gegenwart von Palladiumkatalysatoren umgesetzt werden. Beispiele, die diese Methodik beschreiben, findet man beispielsweise in N. Miyaure, Y. Tanagi, A. Suzuki, Synthetic Communications, 11 (1981) 513, R.F. Heck, Palladium Reagents in Synthesis, Academic Press, London 1985, US 51 30 439 und EP 470 795.

Trotz der Vielzahl von Veröffentlichungen auf dem Gebiet der Synthese von Biphenylen in Gegenwart von Palladiumkatalysatoren sind bisher keine Beispiele für eine größere technische Umsetzung der Methodik bekannt. Dies ist darauf zurückzuführen, daß die beschriebenen Katalysatorsysteme häufig nur mit nicht ökonomischen Ausgangsmaterialien wie Iodaromaten befriedigende Turn Over Zahlen ergeben. Ansonsten müssen bei Bromaromaten und insbesondere bei Chloraromaten generell große Mengen an Katalysator - allgemein > 1 mol% - zugesetzt werden, um technisch nutzbare Umsätze zu erzielen. Aufgrund der Komplexizität der Reaktionsgemische ist zudem kein einfaches Katalysatorrecycling möglich, so daß Katalysatorkosten in der Regel eine technische Realisierung erschweren. Außerdem beobachtet man bei der Suzuki-Kupplung substituierter Biphenyle mit gängigen Katalysatorsystemen wie Pd(OAc)₂/Triphenylphosphan-Gemischen als Nebenreaktion Arylübertragungen aus dem entsprechenden Phosphan (D.F. O'Keefe et al., Tetrahedron Lett., 1992, 6679).

Aus den genannten Gründen ist es von großem industriellem Interesse bessere, technisch nutzbare, Katalysatorsysteme für die Synthese von Biphenylen allgemein und insbesondere für die Arylierung von ökonomisch günstigen Brom-und Chloraromaten und Arylsulfonaten zu finden. Es bestand somit ein großer Bedarf nach einem Verfahren, das die beschriebenen Nachteile vermeidet und Biphenyle in hoher Reinheit in technisch einfacher Weise zugänglich macht.

Diese Aufgabe wird gelöst durch ein Verfahren zur Herstellung von Biphenylen der Formel (I) 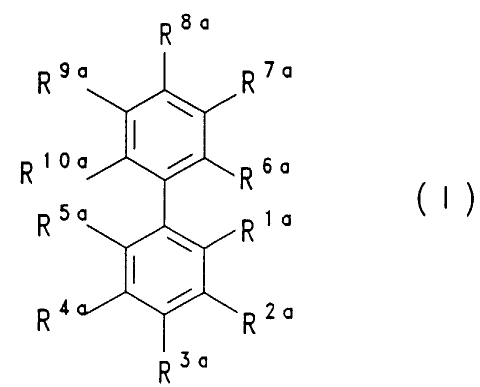 worin R^{1a} bis R^{10a} unabhängig voneinander folgende Bedeutung haben Wasserstoff, C₁-C₁₂-Alkyl, C₁-C₁₂-Alkenyl, C₁-C₁₂-Alkinyl, Alkoxy-(C₁-C₁₂), Acyloxy-(C₁-C₁₂), O-Phenyl, Aryl, Heteroaryl, Fluor, Chlor, OH, NO₂, CN, COOH, CHO, SO₃H, SO₂R, SOR, NH₂, NH-Alkyl-(C₁-C₁₂), N-Alkyl₂-(C₁-C₁₂), C-Hal₃, NHCO-Alkyl-(C₁-C₈), CONH-Alkyl-(C₁-C₄), CON-(Alkyl)₂-(C₁-C₄), COO-Alkyl-(C₁-C₁₂), CONH₂, CO-Alkyl-(C₁-C₁₂), NHCOH, NHCOO-Alkyl-(C₁-C₈), CO-Phenyl, COO-Phenyl, CHCHCO₂-Alkyl-(C₁-C₁₂), CHCHCO₂H, PO-Phenyl₂, PO-Alkyl₂-(C₁-C₈), bedeuten, durch Umsetzung von Halogenaromaten oder Arylsulfonaten der Formel (II) 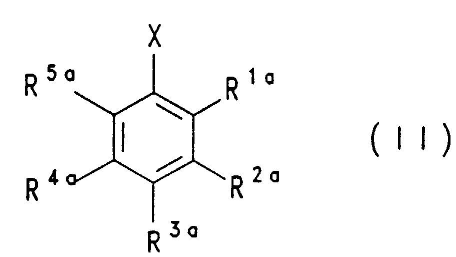 mit Arylborderivaten der Formel III 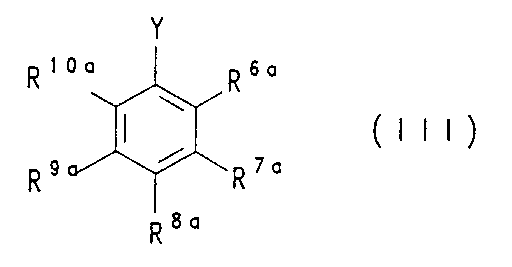 worin R^{1a} bis R^{10a} die angegebene Bedeutung besitzen und X für Brom, Chlor oder OSO₂CF₃, OSO₂-Aryl, OSO₂-Alkyl und Y für B(OH)₂, B(O-Alkyl)₂, B(O-Aryl)₂ steht, dadurch gekennzeichnet, daß man als Katalysator eine Palladiumverbindung der allgemeinen Formel (IV) 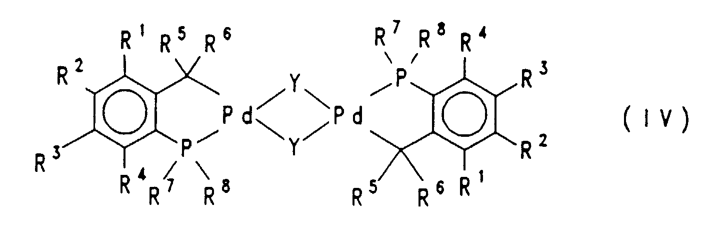 worin
R¹, R², R³, R⁴, R⁵, R⁶ unabhängig voneinander Wasserstoff, (C₁-C₄)-Alkyl, (C₅-C₈)-Cycloalkyl, (C₁-C₄)-Alkoxy, Fluor, NH₂, NH-Alkyl(C₁-C₄), N(Alkyl)₂-(C₁-C₄), CO₂-Alkyl-(C₁-C₄), OCO-Alkyl-(C₁-C₄), oder Phenyl bedeuten, oder R¹ und R², R² und R³, R³ und R⁴, R⁵ und R⁶ zusammen einen aliphatischen oder aromatischen Ring bilden, und R⁷, R⁸ (C₁-C₈)-Alkyl, (C₃-C₁₂)-Cycloalkyl, substituiertes oder unsubstituiertes Aryl sind Y ein Anion einer anorganischen oder organischen Säure bedeutet, einsetzt.

Von Interesse ist das Verfahren zur Herstellung von Verbindungen der Formel (I) worin R^{1a} bis R^{10a} Wasserstoff, C₁-C₈-Alkyl, CN, CO₂H, CHO, COO-Alkyl-(C₁-C₈), Phenyl, 5-Ring-Heteroaryl, 6-Ring-Heteroaryl, CONH₂, CONH-Alkyl-(C₁-C₄), CON-(Alkyl)₂-(C₁-C₄), NO₂, CO-Alkyl-(C₁-C₄), F, Cl, OH, O-Alkyl-(C₁-C₄), NH₂, NHCO-Alkyl-(C₁-C₄) bedeutet.

Wichtig sind auch Verbindungen der Formel (I), worin 4, insbesondere 6, bevorzugt 8, der Substituenten R^{1a} bis R^{10a} Wasserstoff darstellen und die restlichen Substituenten die oben angegebene Bedeutung besitzen.

Von großer Bedeutung ist das Verfahren z.B. zur Herstellung der Verbindungen 4-Alkyl-2'-cyanobiphenyl, 4-Alkyl-2'-biphenylcarbonsäure, 4-Alkyl-2'-biphenylcarbonsäureester, 4-Alkyl-2'-biphenyl-carbonsäureamid, 4-Alkyl-2'-biphenylaldehyd, 4-Alkyl-2'-heteroaryl-biphenyl.

In vielen Fällen haben sich Verbindungen der Formel (IV), worin R¹ bis R⁶ Wasserstoff, Alkyl(C₁-C₄), Phenyl, Cycloalkyl-(C₅-C₈), R⁷ und R⁸ Phenyl, Tolyl, Xylyl, Mesityl, Alkyl(C₁-C₈) und Cycloalkyl(C₅-C₈) bedeuten und Y für Acetat, Propionat, Benzoat, Chlorid, Bromid, Iodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht, bewährt.

Gut geeignet sind z.B. auch Verbindungen der Formel IV worin R¹ - R⁶ für H, (C₁-C₄)-Alkyl, Phenyl und R⁷, R⁸ für (C₁-C₈)-Alkyl, Phenyl, Tolyl, Mesityl und Xylyl stehen.

Sehr gute Ergebnisse liefern die Verbindungen:
trans-Di-µ-acetato-bis[o-(di-o-tolylphosphino)benzyl]dipalladium(II)
trans-Di-µ-chloro-bis[o-(di-o-tolylphosphino)benzyl]dipalladium(II)
trans-Di-µ-bromo-bis[o-(di-o-tolylphosphino)benzyl]dipalladium(II)
trans-Di µ-iodo-bis[o-(di-o-tolylphosphino)benzyl]dipalladium(II)
trans-Di-µ-acetato-bis[o-(dimesitylphosphino)-3,5-dimethylbenzyl]dipalladium(II)
trans-Di-µ-chloro-bis[o-(dimesitylphosphino)-3,5-dimethylbenzyl]dipalladium(II)
trans-Di-µ-bromo-bis[o-(dimesitylphosphino)-3,5-dimethylbenzyl]dipalladium(II)
trans-Di-µ-iodo-bis[o-(dimesitylphosphino)-3,5-dimethylbenzyl]dipalladium(II)
trans-Di-µ-acetato-bis[o-(t-butyl-o-tolylphosphino)benzyl]dipalladium(II)
trans-Di-µ-acetato-bis[o-(di-t-butylphosphino)benzyl]dipalladium(II)
trans-Di-µ-acetato-bis[o-(cyclohexyl-o-tolylphosphino)benzyl]dipalladium(II)

Als Lösungsmittel finden generell inerte organische Lösungsmittel Verwendung. Bevorzugt werden aromatische, dipolar aprotische und polare Lösungsmittel wie Alkyl-, Dialkyl- und Trialkylbenzole, Ether, Ester von aliphatischen Carbonsäuren, Dialkylsulfoxide, N,N-Dialkylamide von aliphatischen Carbonsäuren oder alkylierte Lactame, Amine und Alkohole eingesetzt.

Da bei der Reaktion Halogenwasserstoff abgespalten wird, ist es vorteilhaft diesen durch Zusatz einer Base abzufangen. Dazu geeignet sind primäre, sekundäre oder tertiäre Amine wie Alkylamine, Dialkylamine, Trialkylamine, die alicyclisch oder offenkettig sein können und Alkali- oder Erdalkalisalze von aliphatischen oder aromatischen Carbonsäuren oder der Kohlensäure, wie Natrium-, Kalium-, Calcium-, Magnesiumacetat und entsprechende Carbonate oder Hydrogencarbonate sowie Alkali- oder Erdalkalihydroxide.

Die eingesetzten Palladiumkatalysatoren werden in der Regel vor der eigentlichen Reaktion isoliert synthetisiert, sie können jedoch auch in bestimmten Fällen in situ erzeugt werden ohne Verlust von katalytischer Aktivität.

Das Verfahren wird im allgemeinen bei Temperaturen von 20 bis 200°C durchgeführt. Bewährt haben sich Temperaturen von 60 bis 180°C, insbesondere 100 bis 150°C.

Die Synthese der eingesetzten Palladiumkatalysatoren erfolgt analog zu der deutschen Patentanmeldung Nr. P 4421 753.6.

Die eingesetzten oder sich bildenden Palladacyclen haben in der Regel dimeren Charakter. Bei bestimmten Verbindungen können jedoch auch monomere oder polymere Strukturen vorliegen.

Katalysatoren, die im Rahmen der Umsetzung von Arylhalogeniden mit metallorganischen Arylderivaten verwendet werden, sind generell Palladiumverbindungen. Obwohl sowohl Palladium(II)- als auch Palladium(O)komplexe bei Heck-Reaktionen eingesetzt werden, ist es doch allgemein akzeptiert, daß lediglich Palladium(O)verbindungen die eigentlichen Katalysatoren der Reaktion sind. Dabei formuliert man häufig koordinativ ungesättigte 14-Elektronen Palladium(O)spezies, welche in der Regel mit schwachen Donorliganden wie Phosphanen stabilisiert werden.

Die nachstehenden Beispiele dienen zur Erläuterung des erfindungsgemäßen Verfahrens, ohne es darauf zu beschränken.

### Beispiel 1

165 mmol Brombenzonitril, 247 mmol Phenylboronsäure, 330 mmol Kaliumcarbonat werden mit 0,2 mol% trans-Di-acetato-bis(o-(di-o-tolylphosphino)benzyl)-dipalladium(II) in 300 ml Xylol 16 Stunden auf 130°C erhitzt. Die Reaktionslösung wird nach wäßriger Aufarbeitung destilliert. Ausbeute: 93 % 2-Cyanobiphenyl.

### Beispiel 2

165 mmol Brombenzonitril, 247 mmol 4-Methylphenylboronsäure, 330 mmol Kaliumcarbonat werden mit 0,2 mol% trans-Di-acetato-bis(o-(di-o-tolylphosphino)benzyl)dipalladium(II) in 300 ml Xylol 16 Stunden auf 130°C erhitzt. Die Reaktionslösung wird nach wäßriger Aufarbeitung destilliert. Ausbeute: 94 % 2-Cyano-4-methylbiphenyl.

### Beispiel 3

157 mmol Bromacetophenon, 236 mmol Phenylboronsäure, 314 mmol Kaliumcarbonat werden mit 0,2 mol% trans-Di-acetato-bis(o-(di-o-tolylphosphino)benzyl)dipalladium(ll) in 300 ml Xylol 16 Stunden auf 130°C erhitzt. Die Reaktionslösung wird nach wäßriger Aufarbeitung umkristallisiert. Ausbeute: 94 % 4-Acetylbiphenyl.

### Beispiel 4

55,6 g Chloracetophenon, 1,5 mol eq Phenylboronsäure, 2,0 mol eq Kaliumcarbonat werden mit 0,1 mol% trans-Di-acetato-bis(o-(di-o-tolylphosphino)-benzyl)dipalladium(II) in 300 ml Xylol 16 Stunden auf 130°C erhitzt. Die Reaktionslösung wird nach wäßriger Aufarbeitung umkristallisiert. Ausbeute: 81 % 4-Acetylbiphenyl.

### Beispiel 5

120 mmol 2,4-Dichlor-5-fluorbrombenzol, 180 mmol Phenylboronsäure, 240 mmol Kaliumcarbonat werden mit 0,1 mol% trans-Di-acetato-bis(o-(di-o-tolylphosphino)benzyl)dipalladium(ll) in 300 ml Xylol 16 Stunden auf 130°C erhitzt. Die Reaktionslösung wird nach wäßriger Aufarbeitung umkristallisiert. Ausbeute: 84 % 2,4-Dichlor-5-fluorbiphenyl

### Beispiel 6

157 mmol 4-Fluorbrombenzol, 236 mmol Phenylboronsäure, 314 mmol Kaliumcarbonat werden mit 0,1 mol% trans-Di-acetato-bis(o-(di-o-tolylphosphino)benzyl)dipalladium(II) in 300 ml Xylol 16 Stunden auf 130°C erhitzt. Die Reaktionslösung wird nach wäßriger Aufarbeitung destilliert. Ausbeute: 91 % 4-Fluorbiphenyl.

### Beispiel 7

157 mmol 2-Chlorbenzonitril, 236 mmol 4-Methylphenylboronsäure, 314 mmol Kaliumcarbonat werden mit 0,2 mol% trans-Di-acetato-bis(o-(di-o-tolylphosphino)benzyl-dipalladium(II) in 300 ml Xylol 16 Stunden auf 130°C erhitzt. Die Reaktionslösung wird nach wäßriger Aufarbeitung umkristallisiert. Ausbeute: 73 % 2-Cyano-4-methylbiphenyl

### Beispiel 8 In situ präparierter Katalysator

55,6 g Chloracetophenon, 1,5 mol eq Phenylboronsäure, 2,0 mol eq Kaliumcarbonat werden mit 0,1 mol% Palladiumacetat und 0,1 mol% Tri-o-tolylphosphan in 300 ml Xylol 16 Stunden auf 130°C erhitzt. Die Reaktionslösung wird nach wäßriger Aufarbeitung umkristallisiert. Ausbeute: 65 % 4-Acetylbiphenyl.

### Allgemeine Arbeitsvorschrift für die Beispiele 9 bis 15

100 mmol des entsprechenden Arylhalogenids, 150 mmol Phenylboronsäure und 200 mmol Kaliumcarbonat werden mit 0.1 mmol Palladacyclus in 200 ml Xylol 16 Stunden bei 130°C erhitzt. Anschließend wird die abgekühlte Reaktionsmischung zweimal mit 100 ml Wasser extrahiert und die organische Phase abgetrennt. Das Lösungsmittel wird im Vakuum abgezogen und das Rohprodukt durch Destillation oder Umkristallisation weiter gereinigt.

### Beispiel 9

Arylhalogenid: 2-Brombenzonitril, Katalysator: trans-Di-µ-aceto-bis-[o-(di-o-tolylphosphino)-benzyl]-dipalladium(ll)
Ausbeute: 92 % 2-Cyanobiphenyl

### Beispiel 10

Arylhalogenid: 4-Brombenzaldehyd; Katalysator: trans-Di-µ-aceto-bis[o-(di-t-butylphosphino)-benzyl]-dipalladium(ll)
Ausbeute: 95 % 4-Formylbiphenyl

### Beispiel 11

Arylhalogenid: 4-Brombenzaldehyd; Katalysator: trans-Di-µ-aceto-bis[o-(cyclohexyl-o-tolylphosphino)-benzyl]-dipalladium(II)
Ausbeute: 93 % 4-Formylbiphenyl

### Beispiel 12

Arylhalogenid: 4-Brombenzaldehyd; Katalysator: trans-Di-µ-bromo-bis-[o-(dimesityl-phosphino)-3,5-dimethylbenzyl]-dipalladium(II)
Ausbeute: 98 % 4-Formylbiphenyl

### Beispiel 13

Arylhalogenid: 4-Brombenzaldehyd; Katalysator: trans-Di-µ-bromo-bis-[o-(di-o-tolylphosphino)-benzyl]-dipalladium(II)
Ausbeute: 90 % 4-Formylbiphenyl

### Beispiel 14

Arylhalogenid: 4-Brombenzaldehyd; Katalysator: trans-Di-µ-aceto-bis-[o-(cyclohexyl-o-tolylphosphino)-benzyl]-dipalladium(II)
Ausbeute: 78 % 4-Formylbiphenyl

### Beispiel 15

Arylhalogenid: 2-Fluorbenzol; Katalysator: trans-Di-µ-bromo-bis-[o-(di-o-tolylphosphino)-benzyl]-dipalladium(II)
Ausbeute: 82 % 2-Fluorbiphenyl

### Beispiel 16

15.1 mmol 4-Bromanisol, 22.5 mmol Phenylboronsäure, 33 mmol Kaliumcarbonat werden mit 0.001 mmol-% Bis-(di-o-tolylphosphinobenzyl)-palladiumacetat in 60 ml Xylol bei 140°C bis zum vollständigen Umsatz erhitzt. Nach wäßriger Aufarbeitung und Kristallisation erhält man das Produkt.
Ausbeute: 73 % 4-Methoxybiphenyl

## Patentansprüche

1. Verfahren zur Herstellung von Biphenylen der Formel (I) 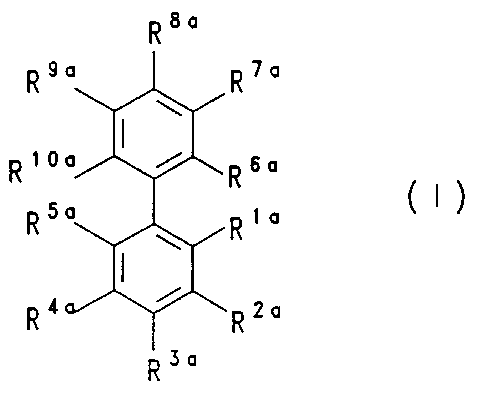 worin R^{1a} bis R^{10a} unabhängig voneinander folgende Bedeutung haben Wasserstoff, C₁-C₁₂-Alkyl, C₁-C₁₂-Alkenyl, C₁-C₁₂-Alkinyl, Alkoxy-(C₁-C₁₂), Acyloxy-(C₁-C₁₂), O-Phenyl, Aryl, Heteroaryl, Fluor, Chlor, OH, NO₂, CN, COOH, CHO, SO₃H, SO₂R, SOR, NH₂, NH-Alkyl-(C₁-C₁₂), N-Alkyl₂-(C₁-C₁₂), C-Hal₃, NHCO-Alkyl-(C₁-C₈), CONH-Alkyl-(C₁-C₄), CON-(Alkyl)₂-(C₁-C₄), COO-Alkyl-(C₁-C₁₂), CONH₂, CO-Alkyl-(C₁-C₁₂), NHCOH, NHCOO-Alkyl-(C₁-C₈), CO-Phenyl, COO-Phenyl, CHCHCO₂-Alkyl-(C₁-C₁₂), CHCHCO₂H, PO-Phenyl₂, PO-Alkyl₂-(C₁-C₈), bedeuten, durch Umsetzung von Halogenaromaten oder Arylsulfonaten der Formel (II) 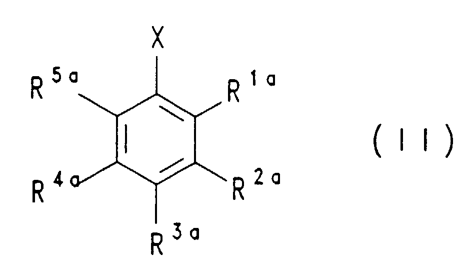 mit Arylborderivaten der Formel III 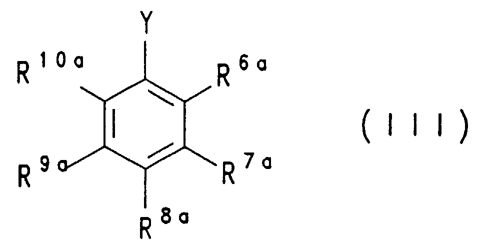 worin R^{1a} bis R^{10a} die angegebene Bedeutung besitzen und X für Brom, Chlor oder OSO₂CF₃, OSO₂-Aryl, OSO₂-Alkyl und Y für B(OH)₂, B(O-Alkyl)₂, B(O-Aryl)₂ steht, dadurch gekennzeichnet, daß man als Katalysator eine Palladiumverbindung der allgemeinen Formel (IV) 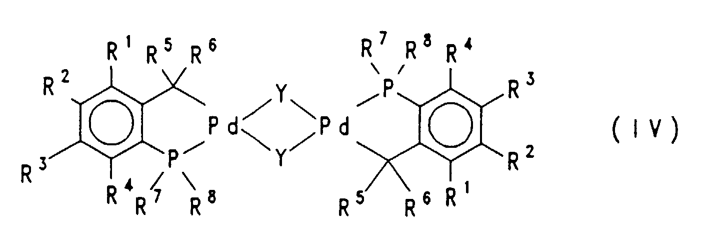 worin
R¹, R², R³, R⁴, R⁵, R⁶ unabhängig voneinander Wasserstoff, (C₁-C₄)-Alkyl, (C₅-C₈)-Cycloalkyl, (C₁-C₄)-Alkoxy, Fluor, NH₂, NH-Alkyl(C₁-C₄), N(Alkyl)₂-(C₁-C₄), CO₂-Alkyl-(C₁-C₄), OCO-Alkyl-(C₁-C₄), oder Phenyl bedeuten, oder R¹ und R², R² und R³, R³ und R⁴, R⁵ und R⁶ zusammen einen aliphatischen oder aromatischen Ring bilden, und
R⁷, R⁸ (C₁-C₈)-Alkyl, (C₃-C₁₂)-Cycloalkyl, substituiertes oder unsubstituiertes Aryl sind
Y ein Anion einer anorganischen oder organischen Säure bedeutet, einsetzt.

2. Verfahren nach Anspruch 1, dadurch gekennzeichnet, daß in Formel (IV) R¹ bis R⁶ unabhängig voneinander Wasserstoff, (C₁-C₄)-Alkyl, (C₅-C₈)-Cycloalkyl, Cycloalkyl,
R⁷, R⁸ Phenyl, Tolyl, Xylyl, Mesityl, Alkyl-(C₁-C₈), Cycloalkyl-(C₅-C₈) bedeuten und
Y für Acetat, Propionat, Benzoat, Chlorid, Bromid, Iodid, Fluorid, Sulfat, Hydrogensulfat, Nitrat, Phosphat, Tetrafluoroborat, Tosylat, Mesylat, Acetylacetonat, Hexafluoracetylacetonat oder Pyrazolyl steht.

3. Verfahren nach Anspruch 1, dadurch gekennzeichnet, daß in Formel (IV) R¹ bis R⁶ für H, (C₁-C₄)-Alkyl, Phenyl, R⁷, R⁸ für (C₁-C₈)-Alkyl, Phenyl, Tolyl, Mesityl und Xylyl stehen.

4. Verfahren nach Anspruch 1, dadurch gekennzeichnet, daß als Katalysator die Verbindungen
trans-Di-µ-acetato-bis[o-(di-o-tolylphosphino)benzyl]dipalladium(II)
trans-Di-µ-chloro-bis[o-(di-o-tolylphosphino)benzyl]dipalladium(II)
trans-Di-µ-bromo-bis[o-(di-o-tolylphosphino)benzyl]dipalladium(II)
trans-Di-µ-iodo-bis[o-(di-o-tolylphosphino)benzyl]dipalladium(II)
trans-Di-µ-acetato-bis[o-(dimesitylphosphino)-3,5-dimethylbenzyl]dipalladium(II)
trans-Di-µ-chloro-bis[o-(dimesitylphosphino)-3,5-dimethylbenzyl]dipalladium(II)
trans-Di-µ-bromo-bis[o-(dimesitylphosphino)-3,5-dimethylbenzyl]dipalladium(II)
trans-Di-µ-iodo-bis[o-(dimesitylphosphino)-3,5-dimethylbenzyl]dipalladium(II)
trans-Di-µ-acetato-bis[o-(t-butyl-o-tolylphosphino)benzyl]dipalladium(II)
trans-Di-µ-acetato-bis[o-(di-t-butylphosphino)benzyl]dipalladium(II)
trans-Di-µ-acetato-bis[o-(cyclohexyl-o-tolylphosphino)benzyl]dipalladium(II)
eingesetzt werden.

5. Verfahren nach mindestens einem der Ansprüche 1 bis 4, dadurch gekennzeichnet, daß der Katalysator in situ hergestellt wird.

6. Verfahren nach mindestens einem der Ansprüche 1 bis 5, dadurch gekennzeichnet, daß R^{1a} bis R^{10a} in Formel (I) Wasserstoff, (C₁-C₈)-Alkyl, CN, CO₂H, CHO, COO-Alkyl-(C₁-C₈), Phenyl, 5-Ring-Heteroaryl, 6-Ring-Heteroaryl, CONH₂, CONH-Alkyl-(C₁-C₄), CON-(Alkyl)₂-(C₁-C₄), NO₂, CO-Alkyl-(C₁-C₄), F, Cl, OH, O-Alkyl-(C₁-C₄), NH₂, NHCO-Alkyl-(C₁-C₄) bedeutet.

7. Verfahren nach mindestens einem der Ansprüche 1 bis 6, dadurch gekennzeichnet, daß 4, insbesondere 6, bevorzugt 8 der Substituenten R^{1a} bis R^{10a} Wasserstoff darstellen.

8. Verfahren nach mindestens einem der Ansprüche 1 bis 6, dadurch gekennzeichnet, daß Formel (I) für 4-Alkyl-2'-cyanobiphenyl, 4-Alkyl-2'-biphenylcarbonsäure, 4-Alkyl-2'-biphenyl-carbonsäureester, 4-Alkyl-2'-biphenyl-carbonsäureamid, 4-Alkyl-2'-biphenyl-aldehyd, 4-Alkyl-2'-heteroaryl-biphenyl steht.

9. Verfahren nach mindestens einem der Ansprüche 1 bis 8, dadurch gekennzeichnet, daß aromatische, dipolar aprotische und polare Lösungsmittel wie Alkyl-, Dialkyl- und Trialkylbenzole, Ether, Ester von aliphatischen Carbonsäuren, Dialkylsulfoxide, N,N-Dialkylamide von aliphatischen Carbonsäuren oder alkylierte Lactame, Amine und Alkohole eingesetzt werden.

10. Verfahren nach mindestens einem der Ansprüche 1 bis 9, dadurch gekennzeichnet, daß die bei der Reaktion entstehende Säure HX durch Zusatz einer Base, insbesondere eines Amins oder eines Alkali oder Erdalkalimetallsalzes einer schwachen Säure abgefangen wird.

11. Verfahren nach Anspruch 10, dadurch gekennzeichnet, daß als Base Alkylamine oder die Carbonate, Hydrogencarbonate oder Acetate von Lithium, Natrium, Kalium, Calcium oder Magnesium eingesetzt werden.

12. Verfahren nach mindestens einem der Ansprüche 1 bis 11, dadurch gekennzeichnet, daß die Reaktion bei Temperaturen von 20 bis 200°C, insbesondere 60 bis 180°C, bevorzugt 100 bis 150°C durchgeführt wird.

## Claims

1. A process for preparing biphenyls of the formula (I) 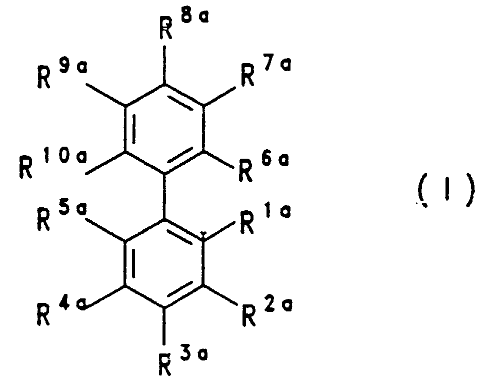 where R^{1a} to R^{10a} are, independently of one another, hydrogen, C₁-C₁₂-alkyl, C₁-C₁₂-alkenyl, C₁-C₁₂-alkynyl, alkoxy-(C₁-C₁₂), acyloxy- (C₁-C₁₂), O-phenyl, aryl, heteroaryl, fluorine, chlorine, OH, NO₂, CN, COOH, CHO, SO₃H, SO₂R, SOR, NH₂, NH-alkyl-(C₁-C₁₂), N-alkyl₂-(C₁-C₁₂), C-Hal₃, NHCO-alkyl-(C₁-C₈), CONH-alkyl-(C₁-C₄), CON-(alkyl)₂-(C₁-C₄), COO-alkyl-(C₁-C₁₂), CONH₂, CO-alkyl-(C₁-C₁₂), NHCOH, NHCOO-alkyl-(C₁-C₈), CO-phenyl, COO-phenyl, CHCHCO₂-alkyl-(C₁-C₁₂), CHCHCO₂H, PO-phenyl₂, PO-alkyl₂-(C₁-C₈), by reaction of haloaromatics or aryl sulfonates of the formula (II) 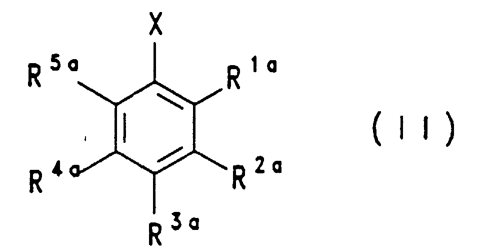 with
arylboron derivatives of the formula III 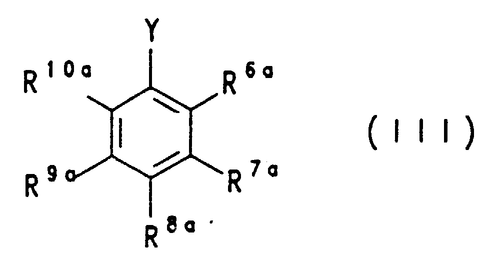 where R^{1a} to R^{10a} are as defined above and X is bromine, chlorine or OSO₂CF₃, OSO₂-aryl, OSO₂-alkyl and Y is B(OH)₂, B(O-alkyl)₂, B(O-aryl)₂, which comprises using as catalyst a palladium compound of the formula (IV) 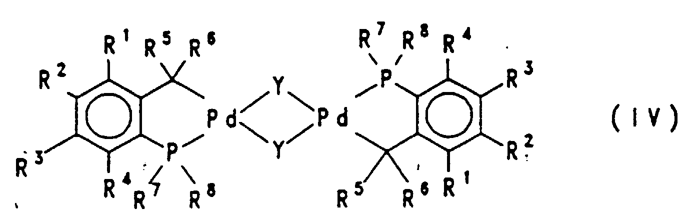 where
R¹, R², R³, R⁴, R⁵, R⁶ are, independently of one another, hydrogen, (C₁-C₄)-alkyl, (C₅-C₈)-cycloalkyl, (C₁-C₄)-alkoxy, fluorine, NH₂, NH-alkyl(C₁-C₄), N(alkyl)₂-(C₁-C₄), CO₂-alkyl-(C₁-C₄), OCO-alkyl-(C₁-C₄), or phenyl, or R¹ and R², R² and R³, R³ and R⁴, R⁵ and R⁶ together form an aliphatic or aromatic ring, and
R⁷, R⁸ are (C₁-C₈)-alkyl, (C₃-C₁₂)-cycloalkyl, substituted or unsubstituted aryl and
Y is an anion of an inorganic or organic acid.

2. The process as claimed in claim 1, wherein, in formula (IV), R¹ to R⁶ are, independently of one another, hydrogen, (C₁-C₄)-alkyl, (C₅-C₈)-cycloalkyl, R⁷, R⁸ are phenyl, tolyl, xylyl, mesityl, alkyl-(C₁-C₈), cycloalkyl-(C₅-C₈) and
Y is acetate, propionate, benzoate, chloride, bromide, iodide, fluoride, sulfate, hydrogensulfate, nitrate, phosphate, tetrafluoroborate, tosylate, mesylate, acetylacetonate, hexafluoroacetylacetonate or pyrazolyl.

3. The process as claimed in claim 1, wherein, in formula (IV), R¹ to R⁶ are H, (C₁-C₄)-alkyl, phenyl, R⁷, R⁸ are (C₁-C₈)-alkyl, phenyl, tolyl, mesityl and xylyl.

4. The process as claimed in claim 1, wherein the catalyst used is one of the compounds
trans-di-µ-acetato-bis[o-(di-o-tolylphosphino)-benzyl]dipalladium(II)
trans-di-µ-chloro-bis[o-(di-o-tolylphosphino)-benzyl]dipalladium(II)
trans-di-µ-bromo-bis[o-(di-o-tolylphosphino)benzyl]-dipalladium(II)
trans-di-µ-iodo-bis[o-(di-o-tolylphosphino)benzyl]-dipalladium(II)
trans-di-µ-acetato-bis[o-(dimesitylphosphino)-3,5-dimethylbenzyl]dipalladium(II)
trans-di-µ-chloro-bis[o-(dimesitylphosphino)-3,5-dimethylbenzyl]dipalladium(II)
trans-di-µ-bromo-bis[o-(dimesitylphosphino)-3,5-dimethylbenzyl]dipalladium(II)
trans-di-µ-iodo-bis[o-(dimesitylphosphino)-3,5-dimethylbenzyl]dipalladium(II)
trans-di-µ-acetato-bis[o-(t-butyl-o-tolylphosphino)-benzyl]dipalladium(II)
trans-di-µ-acetato-bis[o-(di-t-butylphosphino)-benzyl]dipalladium(II)
trans-di-µ-acetato-bis[o-(cyclohexyl-o-tolylphosphino)benzyl]dipalladium(II).

5. The process as claimed in at least one of claims 1 to 4, wherein the catalyst is prepared in situ.

6. The process as claimed in at least one of claims 1 to 5, wherein R^{1a} to R^{10a} in formula (I) are hydrogen, (C₁-C₈)-alkyl, CN, CO₂H, CHO, COO-alkyl-(C₁-C₈), phenyl, 5-ring heteroaryl, 6-ring heteroaryl, CONH₂, CONH-alkyl-(C₁-C₄), CON-(alkyl)₂-(C₁-C₄), NO₂, CO-alkyl-(C₁-C₄), F, Cl, OH, O-alkyl-(C₁-C₄), NH₂, NHCO-alkyl-(C₁-C₄).

7. The process as claimed in at least one of claims 1 to 6, wherein 4, in particular 6, preferably 8, of the substituents R^{1a} to R^{10a} are hydrogen.

8. The process as claimed in at least one of claims 1 to 6, wherein formula (I) is 4-alkyl-2'-cyanobiphenyl, 4-alkyl-2' -biphenylcarboxylic acid, 4-alkyl-2'-biphenylcarboxylic ester, 4-alkyl-2'-biphenylcarboxamide, 4-alkyl-2'-biphenylaldehyde, 4-alkyl-2'-heteroarylbiphenyl.

9. The process as claimed in at least one of claims 1 to 8, wherein aromatic, dipolar aprotic and polar solvents such as alkylbenzenes, dialkylbenzenes and trialkylbenzenes, ethers, esters of aliphatic carboxylic acids, dialkyl sulfoxides, N,N-dialkylamides of aliphatic carboxylic acids or alkylated lactams, amines and alcohols are used.

10. The process as claimed in at least one of claims 1 to 9, wherein the acid HX formed in the reaction is neutralized by adding a base, in particular an amine or an alkali metal or alkaline earth metal salt of a weak acid.

11. The process as claimed in claim 10, wherein the base used is an alkylamine or a carbonate, hydrogencarbonate or acetate of lithium, sodium, potassium, calcium or magnesium.

12. The process as claimed in at least one of claims 1 to 11, wherein the reaction is carried out at temperatures of from 20 to 200°C, in particular from 60 to 180°C, preferably from 100 to 150°C.

## Revendications

1. Procédé de préparation de biphényles de formule I 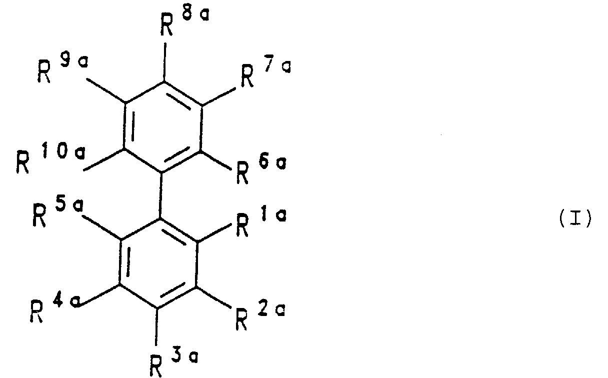 dans laquelle R^{1a} à R^{10a}, indépendamment les uns des autres, représentent les significations suivantes : un atome d'hydrogène, des groupes alkyle en C₁-C₁₂, alcényle en C₁-C₁₂, alcynyle en C₁-C₁₂, (alkoxy en C₁-C₁₂), (acyloxy en C₁-C₁₂), O-phényle, aryle, hétéroaryle, fluor, chlore, OH, NO₂, CN, COOH, CHO, SO₃H, SO₂R, SOR, NH₂, NH-(alkyle en C₁-C₁₂), N-((alkyl)₂ en C₁-C₁₂), C-Hal₃, NHCO-(alkyle en C₁-C₈), CONH-(alkyle en C₁-C₄), CON-((alkyl)₂ en C₁-C₄), COO-(alkyle en C₁-C₁₂), CONH₂, CO-(alkyle en C₁-C₁₂), NHCOH, NHCOO-(alkyle en C₁-C₈), CO-phényle, COO-phényle, CHCHCO₂-(alkyle en C₁-C₁₂), CHCHCO₂H, PO-(phényle)₂, PO-((alkyle)₂ en C₁-C₈), par réaction de composés aromatiques halogénés ou d'arylsulfonates de formule (II) 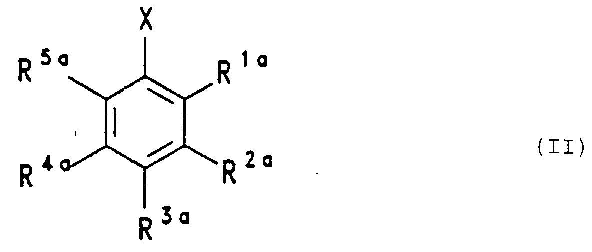 avec des dérivés d'arylbore de formule (III) 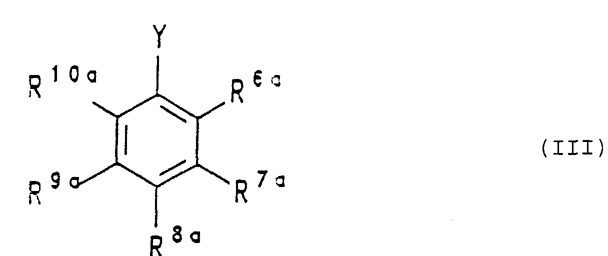 où R^{1a} à R^{10a} ont les significations données et X représente un atome de brome, de chlore ou OSO₂CF₃, OSO₂-aryle, OSO₂-alkyle et Y représente B(OH)₂, B(O-alkyle)₂, B(O-aryle)₂, caractérisé en ce que l'on utilise comme catalyseur un composé de palladium de formule générale (IV) 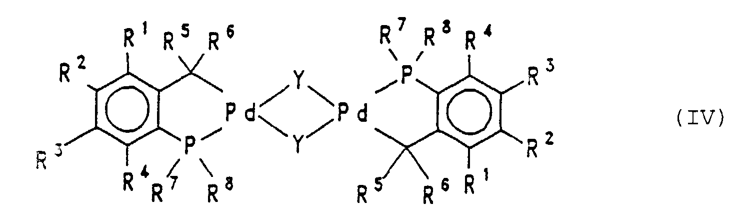 dans laquelle
R¹, R², R³, R⁴, R⁵, R⁶, indépendamment les uns des autres, représentent un atome d'hydrogène, des groupes alkyle en C₁-C₄, cycloalkyle en C₅-C₈, alkoxy en C₁-C₄, fluor, NH₂, NH-(alkyle en C₁-C₄), N((alkyl)₂ en C₁-C₄), CO₂-(alkyle en C₁-C₄), OCO-(alkyle en C₁-C₄), ou phényle, ou R¹ et R², R² et R³, R³ et R⁴, R⁵ et R⁶, ensemble forment un cycle aliphatique ou aromatique, et
R⁷, R⁸ représentent alkyle en C₁-C₈, cycloalkyle en C₃-C₁₂, aryle substitué ou non substitué,
Y représente un anion d'un acide organique ou inorganique.

2. Procédé selon la revendication 1, caractérisé en ce que dans la formule (IV), R¹ à R⁶, indépendamment les uns des autres, représentent un atome d'hydrogène, des groupes alkyle en C₁-C₄, cycloalkyle en C₅-C₈,
R⁷, R⁸ représentent phényle, tolyle, xylyle, mésityle, alkyle en C₁-C₈, cycloalkyle en C₅-C₈, et
Y représente acétate, propionate, benzoate, chlorure, bromure, iodure, fluorure, sulfate, hydrogénosulfate, nitrate, phosphate, tétrafluoroborate, tosylate, mésylate, acétylacétonate, hexafluoroacétylacétonate ou pyrazolyle.

3. Procédé selon la revendication 1, caractérisé en ce que dans la formule (IV), R¹ à R⁶ représentent H, alkyle en C₁-C₄, phényle, R⁷, R⁸ représentent alkyle en C₁-C₈, phényle, tolyle, mésityle et xylyle.

4. Procédé selon la revendication 1, caractérisé en ce que l'on utilise comme catalyseur les composés suivants
trans-di-µ-acétato-bis[o-(di-o-tolylphosphino)benzyl]dipalladium(II),
trans-di-µ-chloro-bis[o-(di-o-tolylphosphino)benzyl]dipalladium(II),
trans-di-µ-bromo-bis[o-(di-o-tolylphosphino)benzyl]dipalladium(II),
trans-di-µ-iodo-bis[o-(di-o-tolylphosphino)benzyl]dipalladium(II),
trans-di-µ-acétato-bis[o-(dimésitylphosphino)-3,5-diméthylbenzyl]dipalladium(II),
trans-di-µ-chloro-bis[o-(dimésitylphosphino)-3,5-diméthylbenzyl]dipalladium(II),
trans-di-µ-bromo-bis[o-(dimésitylphosphino)-3,5-diméthylbenzylbenzyl]dipalladium (II),
trans-di-µ-iodo-bis[o-(dimésitylphosphino)-3,5-diméthylbenzylbenzyl]dipalladium(II),
trans-di-µ-acétato-bis[o-(t-butyl-o-tolylphosphino)-benzyl]di-palladium(II),
trans-di-µ-acétato-bis[o-(di-t-butylphosphino)-benzyl]-dipalladium(II),
trans-di-µ-acétato-bis[o-(cyclohexyl-o-tolylphosphino)-benzyl]dipalladium(II).

5. Procédé selon au moins l'une des revendications 1 à 4, caractérisé en ce que l'on prépare le catalyseur in situ.

6. Procédé selon au moins l'une des revendications 1 à 5, caractérisé en ce que R^{1a} à R^{10a} dans la formule (I) représentent un atome d'hydrogène, des groupes alkyle en C₁-C₈, CN, CO₂H, CHO, COO-(alkyle en C₁-C₈), phényle, hétéroaryle à 5 chaînons, hétéroaryle à 6 chaînons, CONH₂, CONH-(alkyle en C₁-C₄), CON-((alkyl)₂ en C₁-C₄), NO₂, CO-(alkyle en C₁-C₄), F, CI, OH, O-(alkyle en C₁-C₄), NH₂, NHCO-(alkyle en C₁-C₄).

7. Procédé selon au moins l'une des revendications 1 à 6, caractérisé en ce que 4, en particulier 6, de préférence 8 des substituants R^{1a} à R^{10a} représentent un atome d'hydrogène.

8. Procédé selon au moins l'une des revendications 1 à 6, caractérisé en ce que la formule (I) signifie 4-alkyl-2'-cyanobiphényle, acide 4-alkyl-2'-biphénylcarboxylique, esters d'acide 4-alkyl-2'-biphénylcarboxyliques, 4-alkyl-2'-biphénylcarboxamide, 4-alkyl-2 '-biphénylaldéhyde, 4-alkyl-2'-hétéro-aryl-biphényle.

9. Procédé selon au moins l'une des revendications 1 à 8, caractérisé en ce que l'on utilise des solvants aromatiques, dipolaires aprotiques et polaires, tels que des alkyl-, dialkyl- et trialkyl-benzènes, des éthers, des esters d'acides carboxyliques aliphatiques, des dialkylsulfoxydes, des N,N-dialkylamides d'acides carboxyliques aliphatiques ou des amines, des alcools et des lactames alkylés.

10. Procédé selon au moins l'une des revendications 1 à 9, caractérisé en ce que l'on piège l'acide HX formé au cours de la réaction par ajout d'une base, notamment d'une amine ou d'un sel alcalin ou de métaux alcalino-terreux d'un acide faible.

11. Procédé selon la revendication 10, caractérisé en ce que l'on utilise comme base des alkylamines ou des carbonates, des hydrogénocarbonates ou des acétates de lithium, de sodium, de potassium, de calcium ou de magnésium.

12. Procédé selon au moins l'une des revendications 1 à 11, caractérisé en ce que l'on met en oeuvre la réaction à une température de 20 à 200 °C, en particulier de 60 à 180 °C, de préférence de 100 à 150 °C.
